# EUROPEAN PATENT APPLICATION

(11) **EP 2 314 299 A1**
(43) Date of publication of application: **27.04.2011**
(21) Application number: 10192265.6
(22) Date of filing: 29.08.2003
(51) Int. Cl.: A61K 31/5575, A61K 45/06, A61K 31/437, A61K 31/4409, A61K 31/4725, A61K 31/5377, A61K 31/553, A61P 27/06, A61P 43/00

(54) **Therapeutic agent for glaucoma comprising rho kinase inhibitor and prostaglandin**

(30) Priority: 29.08.2002 JP 2002250223
(62) Divisional of application: 03791404.1
(71) Applicant: Santen Pharmaceutical Co., Ltd, Osaka 533-8651 (JP)
(72) Inventor: Nakajima, Tadashi, Nara 630-0101 (JP); Matsugi, Takeshi, Nara 630-0101 (JP); Hara, Hideaki, Nara 630-0101 (JP)
(74) Representative: Peaucelle, Chantal

(57) **Abstract**

A subject of the present invention is to find utility of a combination of a Rho kinase inhibitor and prostaglandins as a therapeutic agent for glaucoma. Actions of reducing intraocular pressure are complemented and/or enhanced each other by combining the Rho kinase inhibitor with prostaglandins. For the administration mode, each drug can be administered in combination or in mixture.

## Description

### Technical Field

The present invention relates to a therapeutic agent for glaucoma comprising the combination of a Rho kinase inhibitor and a prostaglandin.

### Background Art

Glaucoma is an intractable ocular disease with a risk of blindness, involving the increase of intraocular pressure due to various factors and by disordering internal tissues of eyeballs (retina, an optic nerve and the like). A general method of treating glaucoma is intraocular pressure reduction therapy, which is exemplified by pharmacotherapy, laser therapy, surgery therapy and the like.

For the pharmacotherapy, drugs such as sympathomimetic agents (nonselective stimulants such as epinephrine, α ₂ stimulants such as apraclonidine), sympatholytic agents β-blockers such as timolol and befunolol, α ₁-blokers such as bunazosin hydrochloride), parasympathomimetic agents (pilocarpine and the like), carbonic anhydrase inhibitors (acetazolamide and the like) and prostaglandins (isopropyl unoprostone, latanoprost, travoprost, bimatoprost and the like) have been used.

Recently, a Rho kinase inhibitor was found to serve as a therapeutic agent for glaucoma based on a new mechanism of action (WO 00/09162). Invest. Ophthalmol. & Vis. Sci., 42 (1), 137-144 (2001) discloses that the Rho kinase inhibitor increases the aqueous humor outflow from a trabecular meshwork outflow pathway thereby reducing intraocular pressure, and Invest. Ophthalmol. & Vis. Sci., 42 (1), 137-144 (2001) and Invest. Ophthalmol. & Vis. Sci., 42 (5), 1029-1037 (2001) suggest that the mechanism of action is reconstruction of cytoskeleton in trabecular meshwork cells.

Combined use of drugs having actions of reducing intraocular pressure to treat glaucoma has already been studied and there are some reports on the studies. For example, Japanese Patent No. 2726672 reports combined administration of the sympatholytic agent with prostaglandins. WO 02/38158 discloses a method of treating glaucoma by administering some drugs having actions of reducing intraocular pressure in combination to eyes.

However, any reports do not describe the Rho kinase inhibitor at all, and naturally, there is no description concerning advantageous effects brought about by combining the inhibitor with prostaglandins, either.

As mentioned above, neither study nor report has been made concerning therapeutic effects on glaucoma obtained by combining the Rho kinase inhibitor with prostaglandins, so far.

### Disclosure of the Invention

It is a very interesting subject to find utility as a therapeutic agent for glaucoma due to a combination of a Rho kinase inhibitor and a prostaglandin.

Studying precisely effects due to the combination of a Rho kinase inhibitor and a prostaglandin, the present inventors found that an action of reducing intraocular pressure is increased and/or persistence of the action is improved by combining these drugs compared with a case where each drug is used alone and consequently completed the present invention. Detailed test methods and their effects are described later in the section of "Pharmacological Tests". A remarkable increase in action of reducing intraocular pressure and/or remarkable improvement of persistence of the action was observed by combining a Rho kinase inhibitor with a prostaglandin.

The present invention relates to a therapeutic agent for glaucoma comprising the combination of a Rho kinase inhibitor and a prostaglandin. These drugs each other complement and/or enhance their actions.

For the administration mode, each of the Rho kinase inhibitor and the prostaglandin can be in a separate preparation and these drugs can be administered in combination. Alternatively, these drugs can be formulated in a single preparation to be administered. In other words, these drugs can be administered in mixture.

The Rho kinase inhibitors and prostaglandins of the present invention include salts thereof. When these compounds have a basic group such as an amino group, they can be salts with an inorganic acid such as hydrochloric acid or nitric acid or with an organic acid with oxalic acid, succinic acid or acetic acid. When they have an acidic group such as a carboxyl group, they can be salts with an alkali metal such as sodium or potassium or with an alkaline earth metal such as calcium.

The Rho kinase inhibitors and prostaglandins of the present invention include derivatives thereof such as esters. Specific examples of esters are alkyl esters such as methyl esters, ethyl esters and isopropyl esters.

The present invention is characterized by treating glaucoma with the combination of a Rho kinase inhibitor and a prostaglandin.

The Rho kinase inhibitor in the present invention means a compound which inhibits serine/threonine kinase activated with activation of Rho. Examples of Rho kinase inhibitors are the compounds which inhibit ROKα (ROCK-II), p160ROCK (ROK *β ,* ROCK-I) and other compounds which inhibit proteins having a serine/threonine kinase activity. Specific Rho kinase inhibitors are exemplified by Rho kinase inhibitors such as (R)-trans-N-(pyridin-4-yl)-4-(1-aminoethyl)cyclohexanecarboxamide and (R)-(+)-N-(1H-pyrrolo[2,3-b]pyridin-4-yl)-4-(1-aminoethyl)-benzamide disclosed in WO 98/06433 and WO 00/09162, Rho kinase inhibitors such as 1-(5-isoquinolinesulfonyl)homopiperazine and 1-(5-isoquinolinesulfonyl)-2-methylpiperazine disclosed in WO 97/23222 and Nature, 389, 990-994 (1997), Rho kinase inhibitors such as (1-benzylpyrrolidin-3-yl)-(1H-indazol-5-yl)amine disclosed in WO 01/56988, Rho kinase inhibitors such as (1-benzylpiperidin-4-yl)-(1H-indazol-5-yl)amine disclosed in WO 02/100833, Rho kinase inhibitors such as N-[2-(4-fluorophenyl)-6,7-dimethoxy-4-quinazolinyl]-N-(1H-indazol-5-yl)amine disclosed in WO 02/076976, Rho kinase inhibitors such as N-4-(1H-indazol-5-yl)-6,7-dimethoxy-N-2-pyridin-4-yl-quinazolin-2,4-diamine disclosed in WO 02/076977 and Rho kinase inhibitors such as 4-methyl-5-(2-methyl-[1,4]diazepan-1-sulfonyl)isoquinoline disclosed in WO 99/64011.

On the other hand, any prostaglandins having the action of reducing intraocular pressure and utility in treating glaucoma can be used. Prostaglandins having the action of reducing intraocular pressure are specifically exemplified by prostaglandins described in Japanese Laid-open Patent Publication No. 1418/1984 (natural prostaglandins, particularly prostaglandin F2α), prostaglandins such as latanoprost as described in Published Japanese Translation of PCT No. 501025/1991, prostaglandins such as isopropyl unoprostone as described in Japanese Laid-open Patent Publication No. 108/1990, prostaglandins such as bimatoprost as described in Published Japanese Translation of PCT No. 501310/1996, and prostaglandins such as travoprost as described in Japanese Laid-open Patent Publication No. 182465/1998. In particular, latanoprost, isopropyl unoprostone, bimatoprost or travoprost, which has already been on the market as a therapeutic agent of glaucoma, is preferably used.

Examples of glaucoma in the present invention are primary open angle glaucoma, normal intraocular tension glaucoma, hypersecretion glaucoma, ocular hypertension, acute angle-closure glaucoma, chronic closed angle glaucoma, combined-mechanism glaucoma, corticosteroid glaucoma, amyloid glaucoma, neovascular glaucoma, malignant glaucoma, capsular glaucoma, plateau iris syndrome and the like.

To carry out the present invention, preparations can be two preparations prepared by formulating a Rho kinase inhibitor and a prostaglandin separately or one preparation prepared by mixing these ingredients. Particular techniques are unnecessary for the formulation, and the preparations can be prepared using widely-used techniques. A preferred method of administration is eye topical administration, and a preferred dosage form is an ophthalmic solution or an eye ointment.

When a Rho kinase inhibitor and a prostaglandin are formulated in preparations separately, each preparation can be prepared according to known methods. For example, the Rho kinase inhibitor can be formulated in preparations by referring to Formulation Examples described in the above-mentioned International Publications (WO 00/09162 and WO 97/23222). Prostaglandins can be formulated in preparations by referring to Formulation Examples described in the above-mentioned Japanese Laid-open Patent Publications and Published Japanese Translations of PCT (i.e. Japanese Laid-open Patent Publication No. 1418/1984, Published Japanese Translation of PCT No. 501025/1991, Japanese Laid-open Patent Publication No. 108/1990, Published Japanese Translation of PCT No. 501310/1996 and Japanese Laid-open Patent Publication No. 182465/1998), and particularly for latanoprost, isopropyl unoprostone, bimatoprost, travoprost and the like, which have already been on the market as the therapeutic agents for glaucoma, commercially available preparations thereof can be used.

The formulation containing a Rho kinase inhibitor and a prostaglandin in mixture can be also prepared according to known methods. The ophthalmic solutions can be prepared, using isotonic agents such as sodium chloride and concentrated glycerin; buffers such as sodium phosphate buffer and sodium acetate buffer; surfactants such as polyoxyethylene sorbitan monooleate, stearate polyoxyl 40, and polyoxyethylene hardened castor oil; stabilizers such as sodium citrate and sodium edetate; and preservatives such as benzalkonium chloride and paraben, as needed. The pH should be within an ophthalmologically acceptable range and is preferably within a range of pH 4 to pH 8. For reference, a formulation example thereof is described below in the section of Example. However, the formulation example never limits the scope of the invention.

The doses of Rho kinase inhibitor and prostaglandin can be determined depending on the symptom and age of patients, the dosage form, the administration route and the like. The case of instillation is briefly described below. The dose of the Rho kinase inhibitor varies depending on the drug type. The Rho kinase inhibitor can be administered generally within 0.025 to 10,000 µ.g daily from once to several times a day. The dose can be appropriately raised or lowered depending on the age and symptom of patients and the like.

The dose of prostaglandin varies depending on prostaglandin type. The usual daily dose is within a range of 0.1 to 1,000 µ.g, which can be administered from once to several times a day. More specifically, latanoprost and isopropyl unoprostone are generally administered at a daily dose of 1 to 5 µ.g and a daily dose of 30 to 300 µ.g, respectively. Depending on the age and symptom of patients and the like, the doses are varied. Based on similar standards, the doses of the other prostaglandins can be determined.

These doses are also applicable to the administration of the combination of a Rho kinase inhibitor and a prostaglandin. In case that a Rho kinase inhibitor and a prostaglandin are to be administrated in one formulation, the formulation should be prepared by selecting the mixing ratio of two drugs appropriately so that their daily doses might not excess each dose of the separate drugs. The mixed formulation can be administered from once to several times daily.

### Brief Description of Drawings

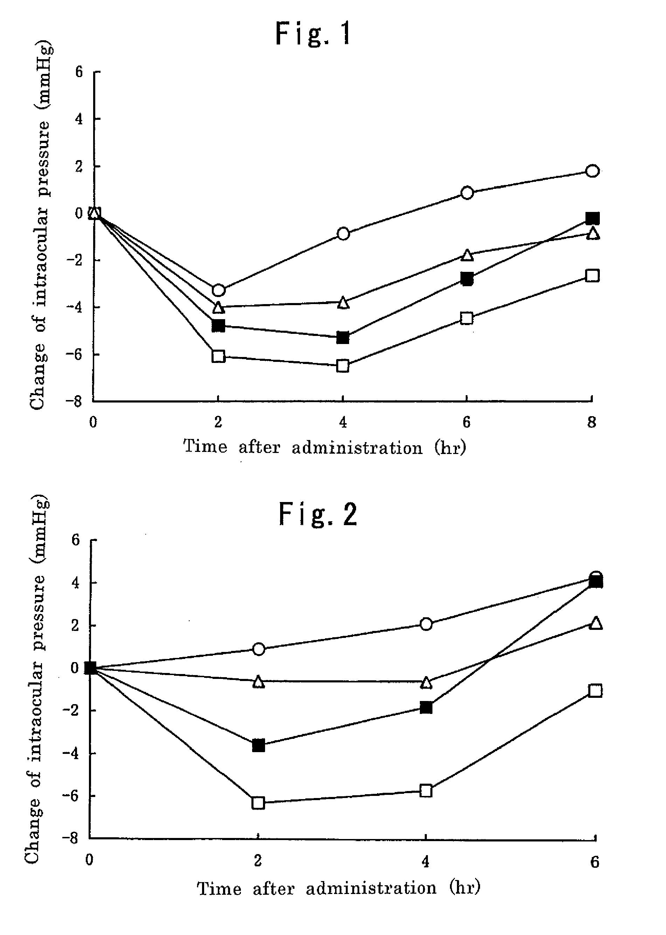

Fig. 1 is a graph showing changes of intraocular pressure with time in respective administration groups. The intraocular pressure is expressed in change from initial intraocular pressure. D represents a Compound A and isopropyl unoprostone combination administration group, ■ represents a single administration group of Compound A, Δ represents a single administration group of isopropyl unoprostone, and O represents a control group.
Fig. 2 is a graph showing changes of intraocular pressure with time in respective administration groups. The intraocular pressure is expressed in change from initial intraocular pressure. D represents a Compound B and isopropyl unoprostone combination administration group, ■ represents a single administration group of Compound B, Δ represents a single administration group of isopropyl unoprostone, and O represents a control group.

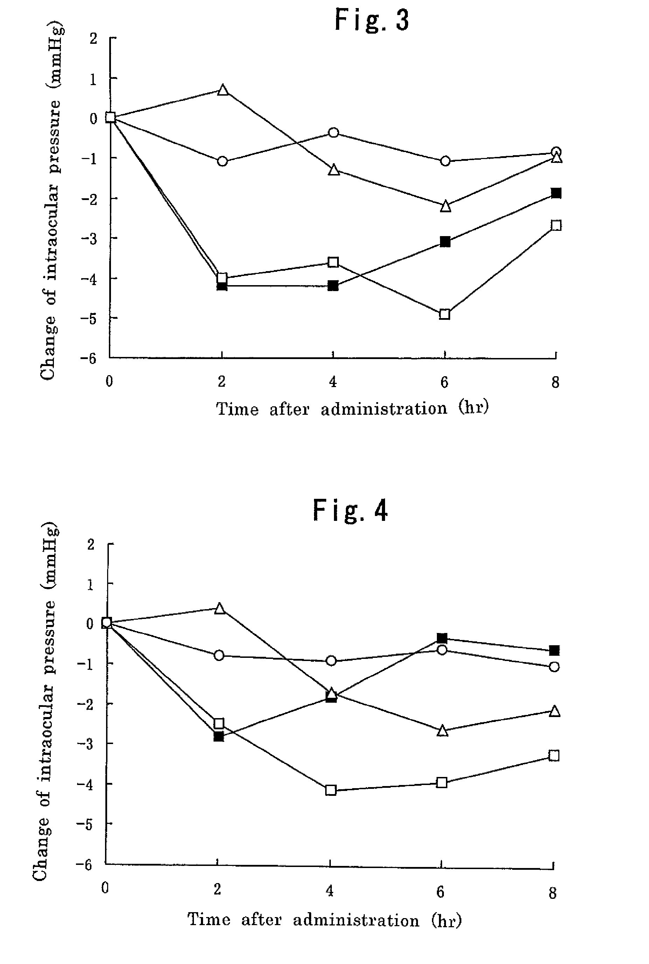

Fig. 3 is a graph showing changes of intraocular pressure with time in respective administration groups. The intraocular pressure is expressed in change from initial intraocular pressure. D represents a Compound A and latanoprost combination administration group, ■ represents a single administration group of Compound A, Δ represents a single administration group of latanoprost, and O represents a control group.
Fig. 4 is a graph showing changes of intraocular pressure with time in respective administration groups. The intraocular pressure is expressed in change from initial intraocular pressure. D represents a Compound B and latanoprost combination administration group, ■ represents a single administration group of Compound B, Δ represents a single administration group of latanoprost, and O represents a control group.

### Best Mode for Carrying out the Invention

A formulation example and pharmacological tests are shown in the following Examples. The Examples are for better understanding of the invention but never limits the scope of the invention.

### Examples

### Formulation Example

A general formulation example of an ophthalmic solution comprising a Rho kinase inhibitor ((R)-(+)-N-(1H-pyrrolo[2,3-b]-pyridin-4-yl)-4-(1-aminoethyl)benzamide dihydrochloride) and prostaglandin (isopropyl unoprostone) in the present invention is shown below.

### Ophthalmic solution (in 100mL)

(R)-(+)-N-(1H-Pyrrolo[2,3-b]pyridin-4-yl)-4-(1-aminoethyl)benzamide

| | |
|---|---|
| dihydrochloride | 0.3 g |
| Isopropyl unoprostone | 0.06 g |
| Boric acid | 0.2 g |
| Concentrated glycerin | 0.25 g |
| Benzalkonium chloride | 0.005 g |
| Diluted hydrochloric acid | quantum sufficient |
| Sodium hydroxide | quantum sufficient |
| Purified water | quantum sufficient |

Ophthalmic solutions having desired combinations and desired concentrations can be prepared by changing the kinds and amounts of Rho kinase inhibitor and prostaglandin and by appropriately changing the amounts of the additives.

### Pharmacological tests

So as to study the utility of the combination of a Rho kinase inhibitor and a prostaglandin, they were administered to Japanese white rabbits (strain: JW, sex: male) or cynomolgus monkeys (Macaca fascicularis, sex: male), examining the effect on reducing intraocular pressure. (R)-(+)-N-(1H-Pyrrolo[2,3-b]pyridin-4-yl)-4-(1-aminoethyl)-benzamide dihydrochloride [Compound A] or 1-(5-isoquinolinesulfonyl)homopiperazine dihydrochloride [Compound B] was used as the Rho kinase inhibitor. Isopropyl unoprostone or latanoprost was used as prostaglandin.

### Preparation of test compound solutions

### 1. Preparation of Rho kinase inhibitor solutions

The Rho kinase inhibitor was dissolved in physiological saline, and then sodium hydroxide was added to the solution to neutralize it (pH 6.0 to 7.0) to thereby prepare Rho kinase inhibitor solutions having desired concentrations.

### 2. Preparation of prostaglandin solutions

A commercially available isopropyl unoprostone ophthalmic solution (trade name: Rescula ophthalmic solution) or a commercially available latanoprost ophthalmic solution (trade name: Xalatan ophthalmic solution) was used as it was, or was diluted with physiological saline to prepare prostaglandin solutions having desired concentrations.

### Method of test

Administering the combination of the Rho kinase inhibitor and prostaglandin, the effect on reducing intraocular pressure was studied. As a reference, administering the Rho kinase inhibitor singly or prostaglandin singly, the effect on reducing intraocular pressure was also studied. As a control, only a vehicle (physiological saline) was administered. As experimental animals, Japanese white rabbits (strain: JW, sex: male) or cynomolgus monkeys (sex: male) were used.

### Method of administration and method of measurement

1) One drop of a 0.4% oxybuprocaine hydrochloride ophthalmic solution was instilled into both eyes of each experimental animal to anesthetize it topically.
2) Intraocular pressure was measured immediately before administering the test compound solution, and the intraocular pressure was referred to as initial intraocular pressure.
3) The Rho kinase inhibitor solution was instilled into one eye of each experimental animal (the other eye was not treated). Since it is impossible to instill the prostaglandin solution at the same time, after a short period (about five minutes), the prostaglandin solution was instilled into the same eye.
4) Two, four, six and eight hours after instilling the Rho kinase inhibitor solution, one drop of the 0.4% oxybuprocaine hydrochloride ophthalmic solution was instilled into both eyes to anesthetize it topically. Then intraocular pressure was measured three times to obtain the average of three measurements.

In Test 2 shown in the following Tests 1-4, intraocular pressure was measured after two, four and six hours.

### 2. Administration of a Rho kinase inhibitor alone

Each test was carried out in the same manner as in the above-mentioned combination administration test except that the prostaglandin solution was replaced with physiological saline.

### 3. Administration of a prostaglandin alone

Each test was carried out in the same manner as in the above-mentioned combination administration test except that the Rho kinase inhibitor solution was replaced with physiological saline.

### 4. Control

Each test was carried out in the same manner as in the above-mentioned combination administration test except that the Rho kinase inhibitor solution and the prostaglandin solution were replaced with physiological saline.

### Tests 1 to 4

The Rho kinase inhibitor solutions, the prostaglandin solutions and the experimental animals to be used in respective tests are shown in Table 1.

Tests 1 to 4 were carried out according to the above-mentioned method of test, and method of administration and method of measurement.

**Table 1**

| | Rho kinase inhibitor solutions | Prostaglandin solutions | Experimental animals |
|---|---|---|---|
| Test 1 | 0.3% Compound A solution (50 *µ*l) | 0.06% Isopropyl unoprostone solution (50 *µ*l) | Rabbit (four rabbits per group) |
| Test 2 | 1% Compound B solution (50 *µl*) | 0.06% Isopropyl unoprostone solution (50 *µ*l) | Rabbit (five rabbits per group) |
| Test 3 | 0.1% Compound A solution (20 *µl*) | 0.005% Latanoprost solution (20 *µ*l) | Cynomolgus monkey (three monkeys per group) |
| Test 4 | 1% Compound B solution (20 *µ*l) | 0.005% Latanoprost solution (20 *µ*l) | Cynomolgus monkey (three monkeys per group) |

### Results and consideration

Results of Test 1, results of Test 2, results of Test 3 and results of Test 4 are shown in Figs. 1, 2, 3 and 4, respectively. Intraocular pressure is expressed in each change from initial intraocular pressure.

As apparent from Figs. 1 to 4, all the Rho kinase inhibitor and prostaglandin combination groups exhibited excellent actions of reducing intraocular pressure compared with administration groups of each drug alone, namely the Rho kinase inhibitor administration groups or the prostaglandin administration groups, and exhibited improvement of persistence of the actions. The above-mentioned results show that a stronger reducing effect on intraocular pressure and/or improvement of persistence is obtained by combining the Rho kinase inhibitor with prostaglandins.

### Industrial Applicability

An action on reducing intraocular pressure is increased and/or persistence of the action is improved by administering a Rho kinase inhibitor in combination with a prostaglandin to eyes. Accordingly, the combination is useful as a therapeutic agent for glaucoma.

## Claims

1. A therapeutic agent for glaucoma comprising a combination of a Rho kinase inhibitor and a prostaglandin wherein the Rho kinase inhibitor is (R)-trans-N-(pyridin-4-yl)-4-(1-aminoethyl)cyclohexanecarboxamide, (R)-(+)-N-(1H-pyrrolo[2,3-b]pyridin-4-yl)-4-(1-aminoethyl)benzamide, 1-(5-isoquinolinesulfonyl)-homopiperazine or 1-(5-isoquinolinesulfonyl)-2-methylpiperazine, and the prostaglandin is isopropyl unoprostone, latanoprost, travoprost or bimatoprost, with the proviso that the Rho kinase is not (R)-(+)-N-(1H-pyrrolo[2,3-b]pyridin-4-yl)-4-(1-aminoethyl) benzamide when the prostaglandin is latanoprost.

2. The therapeutic agent of claim 1, wherein the combination of said Rho kinase inhibitor and said prostaglandin complement and/or enhance their actions each other.

3. Use of a combination of a Rho kinase inhibitor and a prostaglandin in the manufacture of a therapeutic agent for glaucoma, wherein the Rho kinase inhibitor is (R)-trans-N-(pyridin-4-yl)-4-(1-aminoethyl)cyclohexanecarboxamide, (R)-(+)-N-(1H-pyrrolo[2,3-b]pyridin-4-yl)-4-(1-aminoethyl)benzamide, 1-(5-isoquinolinesulfonyl)-homopiperazine or 1-(5-isoquinolinesulfonyl)-2-methylpiperazine, and the prostaglandin is isopropyl unoprostone, latanoprost, travoprost or bimatoprost, with the proviso that the Rho kinase is not (R)-(+)-N-(1H-pyrrolo[2,3-blpyridin-4-yl)-4-(l-aminoethyl) benzamide when the prostaglandin is latanoprost.

4. The use of claims 3, wherein the combination of said Rho kinase inhibitor and said prostaglandin complement and/or enhance their actions each other.
